# EUROPÄISCHE PATENTSCHRIFT

(11) **EP 0 794 941 B1**
(45) Veröffentlichungstag und Bekanntmachung des Hinweises auf die Patenterteilung: **19.05.1999**
(21) Anmeldenummer: 95940223.1
(22) Anmeldetag: 21.11.1995
(51) Int. Cl.: C07C 249/12

(54) **VERFAHREN ZUR HERSTELLUNG VON OXIMETHERN DURCH UMSETZUNG VON OXAMIN MIT DIALKYLCARBONATEN**
PROCESS FOR PREPARING OXIME ETHERS BY REACTING OXAMINE WITH DIALKYLCARBONATES
PROCEDE DE PREPARATION D'ETHERS D'OXIME PAR REACTION D'OXAMINE AVEC DES CARBONATES DE DIALKYLE

(30) Priorität: 01.12.1994 DE 4442730
(43) Veröffentlichungstag der Anmeldung: 17.09.1997
(73) Patentinhaber: BASF AKTIENGESELLSCHAFT, 67056 Ludwigshafen (DE)
(72) Erfinder: WINGERT, Horst, D-68159 Mannheim (DE); KEIL, Michael, D-67251 Freinsheim (DE)
(86) Internationale Anmeldenummer: EP9504580
(87) Internationale Veröffentlichungsnummer: WO9616932

(56) Entgegenhaltungen:
- EP-A- 0 554 767
- JOURNAL OF ORGANIC CHEMISTRY, Bd. 58, Nr. 21, 1993 EASTON US, Seiten 5765-70, C. A. MARQUES ET AL. 'Reaction of oximes with dimethyl carbonate: a new entry to 3-methyl-4,5-disubstituted-4-oxazolin-2-on es'
- ACS SYMPOSIUM SERIES , Bd. 443, 1991 Seiten 226-235, XP 000563581 'Synthesis of the new graminicide propaquizafop'

## Beschreibung

Die vorliegende Erfindung betrifft ein Verfahren zur Herstellung von Oximethern der allgemeinen Formel Ia, 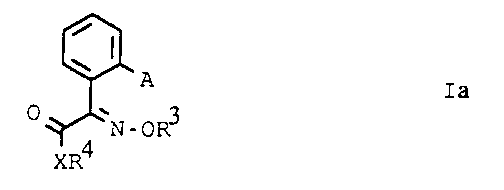 wobei
- R³, R⁴: gleich oder verschieden C₁-C₆-Alkyl und R⁴ zusätzlich Wasserstoff bedeuten und
- X: Sauerstoff und NH bedeuten und
- A: für die folgenden Reste steht: CH_{3,} O-Aryl, CH₂-O-Aryl, 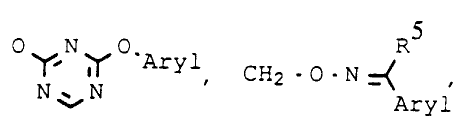 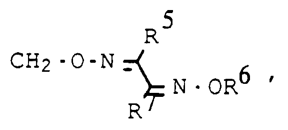 wobei
- R⁵-R⁷: gleich oder verschieden Wasserstoff, C₁-C₄-Alkyl, Aryl und Heteroaryl bedeutet, mit der Maßgabe, daß
Aryl
für Phenyl und Naphthyl steht und durch einen bis zu drei der folgenden Reste substituiert sein können: Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoximino-C₁-C₄-alkyl, Aryl, Aryloxy, Benzyl, Benzyloxy, Heteroaryl, Heteroaryloxy, C₃-C₆-Cycloalkyl, C₁-C₄-Dialkylamino, CO₂CH₃, CO₂C₂H₅, Formyl und Acetyl
und daß Heteroaryl
für einen ggf. substituierten aromatischen Fünfring- oder Sechsring-Heterocyclus steht,
indem man ein Oxim der allgemeinen Formel IIa 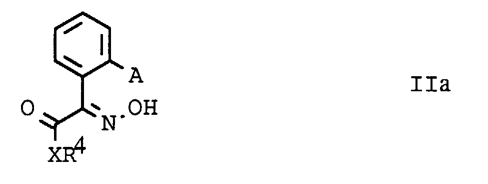 in der die Substituenten die o. a. Bedeutung haben, gegebenenfalls in Gegenwart eines organischen Verdünnungsmittels mit einer Base in das entsprechende Salz überführt und dieses mit einem Dialkylcarbonat der allgemeinen Formel III umsetzt, 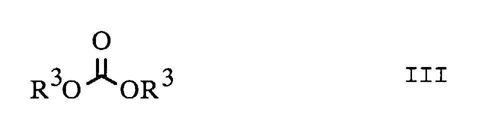 wobei R³ die o. a. Bedeutung hat.

Es ist aus Houben-Weyl, Methoden der organischen Chemie, 4. Auflage Bd. 10/4, S. 7ff bekannt, Oximether der allgemeinen Formel I im wesentlichen auf zweierlei Weise herzustellen:
a) durch direkte Oximierung einer Carbonyl-Vorstufe mit Alkoxyamin-salzen, oder 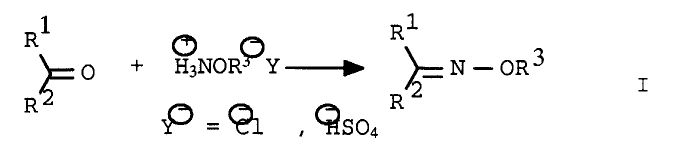
b) durch Alkylierung einer Oxim-Vorstufe II mit einem Alkylierungsmittel R³⁻Z wie z. B. Dialkylsulfat oder Alkylhalogenid

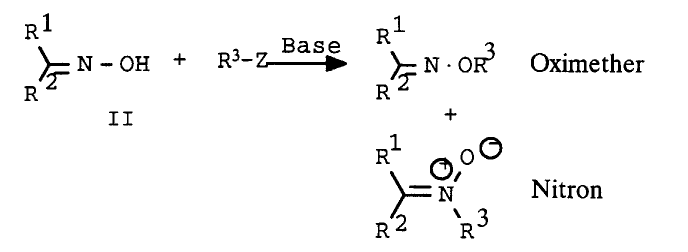

Beide Verfahrensvarianten weisen jedoch erhebliche Nachteile auf, die eine großtechnische Herstellung der Verbindungen der allgemeinen Formel I erschweren. Bei der Verfahrensvariante a) ist vor allem der hohe Preis sowie die schlechte Verfügbarkeit von Alkoxyaminsalzen zu nennen.

Der hauptsächliche Nachteil der Variante b) ist die geringe Selektivität der Alkylierungsreaktion; so erhält man neben dem gewünschten O-Alkylierungsprodukt auch in 10 - 20 % Ausbeute das N-Alkylierungsprodukt in Form des entsprechenden Nitrons.

Beispielsweise werden in der EP-B 253 213 diese beiden Herstellweisen zur Herstellung von neuen Oximethern beschrieben.

In der EP-A 554 767 wird ein Verfahren zur Herstellung von E-Oximethern von Phenylglyoxylsäureestern beschrieben, bei dem man E-Oxime von Phenylglyoxylsäureestern mit einem Alkylierungsmittel umsetzt, wobei man als Nebenprodukt auch das entsprechende unter Verfahrensweise b) beschriebene Nitron erhält.

Aus J. Org. Chem. 1993, 58, 5765-70, ist bekannt, daß sich aliphatische Oxime mit Dimethylcarbonat im wesentlichen zu Oxazolinonen umsetzen und nur in untergeordnetem Maße zu O-Methylderivaten. Aus der Umsetzung von Benzophenonoxim mit Dimethylcarbonat resultierte 56 % des Oximmethylethers und 24 % des N-Methylnitrons. Aus der Umsetzung von Acetophenon mit Dimethylcarbonat resultierte 45 % des korrespondierenden O-Methylderivates. Dieses Dokument zeigt, daß die Umsetzung von Oximen mit Dimethylcarbonat eine geringe Selektivität in Bezug auf die Bildung des Oximethers aufweist.

Der vorliegenden Erfindung lag daher die Aufgabe zugrunde, ein einfaches, kostengünstiges und großtechnisch anwendbares Verfahren zur Herstellung der Oximether Ia zu finden.

Wie wir überraschend fanden, läßt sich ein Oxim der allgemeinen Formel IIa mit einem Dialkylcarbonat in Gegenwart einer Base sehr selektiv und in gewünschter Weise am O-Atom alkylieren. Das unerwünschte Nebenprodukt, das durch Alkylierung am Oxim-stickstoff-Atom gebildet wird (Nitron) entsteht bei dem vorliegenden Verfahren in max. 5 % Ausbeute, in der Regel jedoch in < 2 % Ausbeute.

Es ist zwar prinzipiell bekannt, daß man Dialkylcarbonate als Alkylierungsagentien einsetzen kann, jedoch war es völlig überraschend, daß sich damit Oxime der allgemeinen Formel IIa überhaupt und mit dieser hohen O-Selektivität alkylieren lassen.

Bei dem erfindungsgemäßen Verfahren geht man in der Regel so vor, daß man ein Oxim der allgemeinen Formel IIa zunächst mit einer Base umsetzt und das gebildete Oximat anschließend mit Dialkylcarbonat, bei Temperaturen von 80 - 130°C, vorzugsweise bei der Siedetemperatur des jeweiligen Dialkylcarbonats zur Reaktion bringt.

Oft ist es von Vorteil das gebildete Oximat zu isolieren und dieses nachfolgend in reiner Form mit einem Dialkylcarbonat, vorzugsweise mit Dimethylcarbonat bei Temperaturen von 80 - 130°C, vorzugsweise bei der jeweiligen Siedetemperatur des entsprechenden Dialkylcarbonats umzusetzen. Vorzugsweise verzichtet man hierbei auf die Verwendung eines zusätzlichen Lösungs- oder Verdünnungsmittels und setzt stattdessen das Dialkylcarbonat im Überschuß ein. Dieses kann während oder nach Abschluß der Reaktion durch Destillation sowohl im Vakuum, wie auch unter Normaldruck zurückgewonnen werden.

Möchte man die Umsetzung dennoch in Gegenwart eines Verdünnungsmittels durchführen, so bietet sich die Verwendung von Toluol, Xylol, Dimethylformamid oder einem Alkohol, wie z. B. Methanol oder Ethanol an.

Die Überführung der Oxime in die Oximate erfolgt durch Reaktion mit einer organischen oder anorganischen Base. Geeignete organische Basen sind tertiäre Amine wie Trialkylamine. Beispiele für Trialkylamine sind Triethylamin, Trimethylamin und Diethylmethylamin.

Geeignete anorganische Basen sind Alkalihydroxide, -carbonate, -alkoholate oder -hydride wie beispielsweise Kaliumcarbonat, Kaliumhydroxid, Kaliummethanolat, Kalium-tert.-butylat, Natriumcarbonat, Natriumhydroxid, Natriummethanolat und Natriumhydrid. Bevorzugt ist Triethylamin, Natriummethanolat oder Kaliumcarbonat. Die Umsetzung der Oxime mit einer Base wird vorzugsweise in Gegenwart eines Verdünnungsmittels durchgeführt, wie z.B. Toluol, Xylol, Methanol, Ethanol oder Dimethylcarbonat. Bevorzugt sind Methanol und Dimethylcarbonat. Diese Umsetzung wird bei Temperaturen im Bereich von + 20°C bis + 100°C durchgeführt. Die Base wird mit 0,1 bis 3 Moläquivalente eingesetzt, bezogen auf Oxim.

Als Dialkylcarbonate lassen sich C₁-C₆-Dialkylcarbonate einsetzen wie z. B. Dimethylcarbonat, Diethylcarbonat, Di-n-propylcarbonat, Di-iso-propylcarbonat, Di-n-butylcarbonat, Di-n-pentyl-carbonat, Di-n-hexyl-carbonat; bevorzugt werden allerdings die Reaktionen mit Dimethylcarbonat. Benutzt man ein zusätzliches Lösungs- oder Verdünnungsmittel, so setzt man 1 bis 10 Moläquivalente Dialkylcarbonat ein. Vorzugsweise benutzt man jedoch das Dialkylcarbonat ohne zusätzliches Verdünnungsmittel im Überschuß.

Die Umsetzung der Oximate mit den Dialkylcarbonaten erfolgt in der Regel bei Normaldruck in einem Temperaturbereich von 80 - 130°C. Es kann jedoch von Vorteil sein, die Umsetzung in einem Autoklaven in einem Druckbereich von 1 - 100 bar, vorzugsweise im Bereich von 1 bis 20bar durchzuführen. In diesem Druckbereich wird die Base bevorzugt im Bereich von 0,1 bis 1 Moläquivalent, bezogen auf Oxim, eingesetzt.

Das erfindungsgemäße Verfahren läßt sich zur Herstellung der Oximether der Formeln Ia anwenden, in denen die Substituenten die folgende Bedeutung haben:

Alkyl steht für gesättigte, geradkettige oder verzweigte Kohlenwasserstoffreste mit 1 bis 6 Kohlenstoffatomen, z. B. Methyl, Ethyl, Propyl, 1-Methylethyl, Butyl, 1-Methyl-propyl, 2-Methylpropyl, 1-1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,-2-Trimethylpropyl, 1,2,2-Trimethylpropyl, 1-Ethyl-1-methylpropyl und 1-Ethyl-2-methylpropyl;

Halogen steht für Fluor, Chlor, Brom und Jod.

Halogenalkyl steht für geradkettige oder verzweigte Alkylgruppen mit 1 bis 4 Kohlenstoffatomen wie z.B. Methyl, Ethyl, Propyl, 1-Methylethyl, Butyl, 1-Methyl-propyl, 2-Methylpropyl, 1-1-Dimethylethyl, wobei in diesen Gruppen teilweise oder vollständig die Wasserstoffatome durch Halogenatome ersetzt sein können, z. B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluor-ethyl, 2-Chlor-2,2-difluorethyl, 2,2-Dichlor-2-fluorethyl, 2,2,2-Trichlorethyl und Pentafluorethyl;
Alkoxy steht für geradkettige oder verzweigte Alkylgruppen mit 1 bis 4 Kohlenstoffatomen Methyl, Ethyl, Propyl, 1-Methylethyl, Butyl, 1-Methyl-propyl, 2-Methylpropyl, 1-1-Dimethylethyl, welche über ein Sauerstoffatom (-O-) an das Gerüst gebunden sind;
Halogenalkoxy steht für geradkettige oder verzweigte Halogenalkylgruppen mit 1 bis 4 Kohlenstoffatomen wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluor-ethyl, 2-Chlor-2,2-difluorethyl, 2,2-Dichlor-2-fluorethyl, 2,2,2-Trichlorethyl und Pentafluorethyl; welche über eine Sauerstoffatom (-O-) an das Gerüst gebunden sind;
Alkoximino-alkyl steht für wie z. B. Methoximino-methyl, 1-Methoximino-1-ethyl, Ethoximino-methyl, 1-Methoximino-2-ethyl, 1-Ethoximino-1-ethyl, 1-Ethoximino-2-ethyl;
Cycloalkyl steht für monocyclische Alkylgruppen mit 3 bis 6 Kohlenstoffringgliedern, z. B. C₃-C₆-Cycloalkyl wie Cyclopropyl, Cyclobutyl, Cyclopentyl, Cyclohexyl;

Hetaryl bzw. Hetaryloxy steht für aromatisch mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder kein Stickstoffatom und ein Sauerstoff- oder ein Schwefelatom enthalten können und welche direkt (Hetaryl) oder über ein Sauerstoffatom (Hetaryloxy) an das Gerüst gebunden sind, z. B.
- 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z. B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend ein bis vier Stickstoffatome oder ein bis drei Stickstoffatome und ein Schwefel- oder Sauerstoffatom oder kein Stickstoffatom und ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder kein Stickstoffatom und ein Sauerstoff- oder Schwefelatom als Ringglieder enthalten können, z. B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-oxadiazol-2-yl, 1,3,4-Thiadiazol-2-yl, 1,3,4-Triazol-2-yl;
- benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome:
   5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können, wobei diese Ringe über eines der Stickstoffringglieder an das Gerüst gebunden sind;
- 6-gliedriges Heteroaryl, enthaltend ein bis drei bzw. ein bis vier Stickstoffatome:
   6-Ring Heteroarylgruppen, welche neben Kohlenstoffatomen ein bis drei bzw. ein bis vier Stickstoffatome als Ringglieder enthalten können, z. B. 2-Pyridinyl, 3-Pyridinyl,
   4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend ein bis vier Stickstoffatome:
   6-Ring Heteroarylgruppen in welchen zwei benachbarte Kohlenstoffringglieder durch ein Buta-1,3-dien-1,4-diylgruppe verbrückt sein können, z. B. Chinolin, Isochinolin, Chinazolin und Chinoxalin.

Die Oximether der allgemeinen Formel Ia bzw. deren Vorstufen, die Oxime der allgemeinen Formel IIa können als E- und als Z-Isomer auftreten. Beide Isomeren wie auch E/Z-Isomerengemische werden von der Anmeldung gleichermaßen erfaßt. Bevorzugt setzt man jedoch die Oxime der allgemeinen Formel IIa als E-Isomere ein und erhält, nach Umsetzung gemäß Anspruch 1 die E-Oximether der allgemeinen Formel Ia.

Im Folgenden wird das erfindungsgemäße Verfahren an Hand von Beispielen vorgestellt.

### Beispiel 1

Herstellung von E-2-(2'-Methylphenoxymethyl)-Phenylglyoxylsäuremethylester-O-methyloxim.

Zu 18 g einer 30 %igen Natriummethanolat/Methanol-Lösung gibt man bei Raumtemperatur eine Lösung von 30 g E-2-(2'-Methylphenoxymethyl)-phenylglyoxylsäuremethylester-Oxim [bekannt aus EP 554767] [Reinheit: 95,6 %ig] und engt anschließend am Rotationsverdampfer bei 50°C/1,33 kPa (10 mm) bis zur Trockne ein. Man gibt 100 ml Dimethylcarbonat zu und erhitzt die Suspension 11 Stunden unter Rückfluß. Nach dem Abkühlen auf Raumtemperatur gibt man 200 ml Wasser zu und extrahiert zweimal mit je 150 ml Methylenchlorid. Die vereinigten organischen Phasen werden getrocknet und eingeengt. Der verbleibende Rückstand (29,5 g) weist die folgende Zusammensetzung auf [Gew.-% -quantitative HPLC-Analytik].

| | |
|---|---|
| 1,2 % | Ausgangsmaterial (E-Oxim) |
| 0,5 % | 2-(2'-Methylphenoxymethyl)-phenylglyoxylsäuremethylestermethylnitron (Nitron) |
| 88,7 % | E-2-(2'-Methylphenoxymethyl)-phenylglyoxylsäuremethylester-O-methyloxim (E-Oximether) |
| 1,2 % | 2-(2'-Methylphenoxymethyl)-benzonitril. |

Die Selektivität bezüglich O/N-Alkylierung ist 177 : 1, bei einer Ausbeute von 86,7 % (Nitron + E-Oximether).

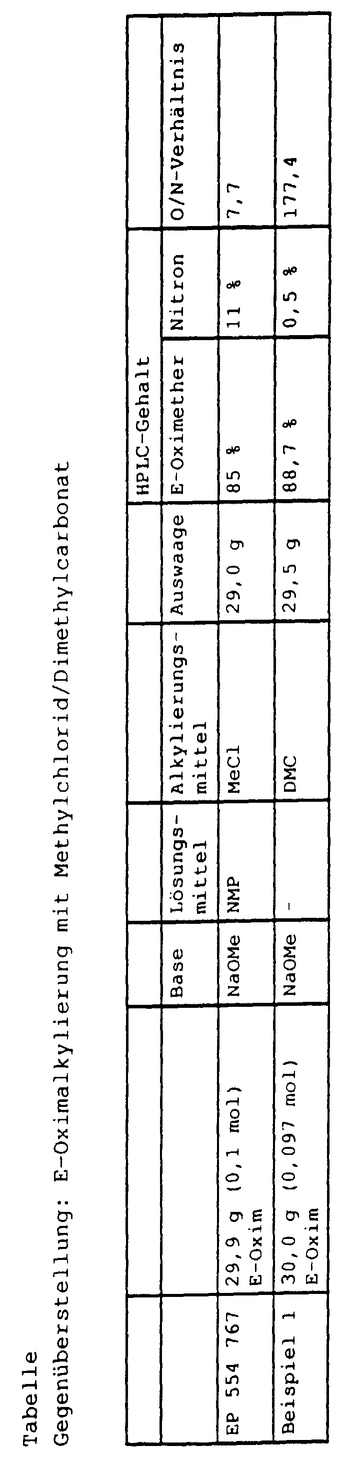

## Patentansprüche

1. Verfahren zur Herstellung von Oximethern der allgemeinen Formel Ia, 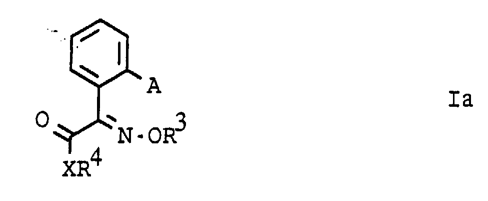 wobei
R³, R⁴ gleich oder verschieden C₁-C₆-Alkyl und R⁴ zusätzlich Wasserstoff bedeuten und
X Sauerstoff und NH bedeuten und
A für die folgenden Reste steht: CH_{3,} O-Aryl, CH₂-O-Aryl, 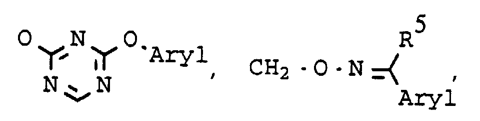 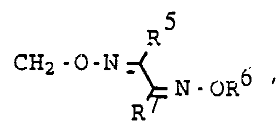 wobei
R⁵-R⁷ gleich oder verschieden Wasserstoff, C₁-C₄-Alkyl, Aryl und Heteroaryl bedeutet, mit der Maßgabe, daß
Aryl
für Phenyl und Naphthyl steht und durch einen bis zu drei der folgenden Reste substituiert sein können: Halogen, Cyano, Nitro, C₁-C₄-Alkyl, C₁-C₄-Alkoxy, C₁-C₄-Halogenalkyl, C₁-C₄-Halogenalkoxy, C₁-C₄-Alkoximino-C₁-C₄-alkyl, Aryl, Aryloxy, Benzyl, Benzyloxy, Heteroaryl, Heteroaryloxy,
C₃-C₆-Cycloalkyl, C₁-C₄-Dialkylamino, CO₂CH₃, CO₂C₂H₅, Formyl und Acetyl
und daß Heteroaryl
für einen ggf. substituierten aromatischen Fünfring- oder Sechsring-Heterocyclus steht,
dadurch gekennzeichnet, daß man ein Oxim der allgemeinen Formel IIa 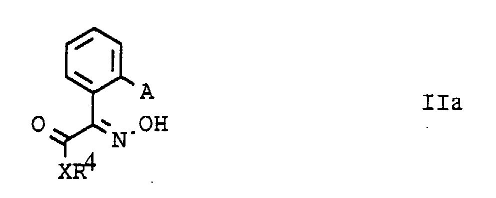 in der die Substituenten die o. a. Bedeutung haben, gegebenenfalls in Gegenwart eines organischen Verdünnungsmittels mit einer Base in das entsprechende Salz überführt und dieses mit einem Dialkylcarbonat der allgemeinen Formel III umsetzt, 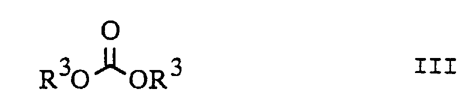 wobei R³ die o. a. Bedeutung hat.

2. Verfahren gemäß Anspruch 1, dadurch gekennzeichnet, daß man ein Oxim der allgemeinen Formel IIb, in der die Substituenten die o.a. Bedeutung haben, 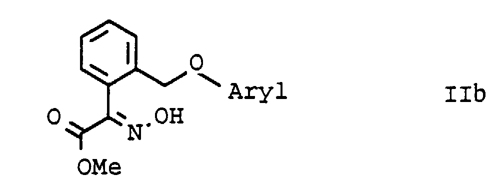 zuerst mit Natriummethanolat in das entsprechende Natriumsalz überführt und dieses dann mit Dimethylcarbonat zu dem entsprechenden Oximether umsetzt.

3. Verfahren gemäß Anspruch 1, dadurch gekennzeichnet, daß man die Verbindung IIc 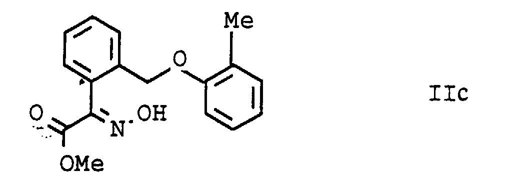 mit Natriummethanolat in das entsprechende Natriumsalz überführt und dieses anschließend mit Dimethylcarbonat zu dem Oximether Ib umsetzt.

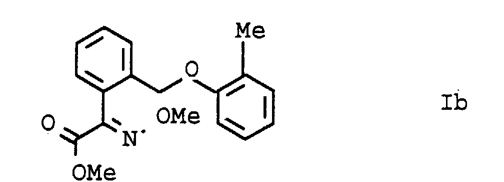

4. Verfahren gemäß Anspruch 1, dadurch gekennzeichnet, daß R³ und R⁴ Methyl bedeuten und X für Sauerstoff steht.

5. Verfahren gemäß Anspruch 1, dadurch gekennzeichnet, daß man als Base ein tertiäres Amin oder ein Alkali-hydroxid, -carbonat, -alkoholat oder -hydrid verwendet.

6. Verfahren gemäß Anspruch 5, dadurch gekennzeichnet, daß man als Base Triethylamin, Trimethylamin oder Diethylmethylamin verwendet.

7. Verfahren gemäß einem der Ansprüche 1 bis 6, dadurch gekennzeichnet, daß man 0, 1 bis 3 Moläquivalente Base einsetzt.

8. Verfahren gemäß einem der Ansprüche 1 bis 7, dadurch gekennzeichnet, daß man als Dialkylcarbonat Dimethylcarbonat, Diethylcarbonat, Di-n-propylcarbonat, Di-iso-propylcarbonat, Di-n-butylcarbonat, Di-n-pentyl-carbonat oder Di-n-hexyl-carbonat verwendet.

9. Verfahren gemäß einem der Ansprüche 1 bis 8, dadurch gekennzeichnet, daß man die Reaktion des Oxims mit der Base bei einer Temperatur von 20 bis 100°C durchführt.

10. Verfahren gemäß einem der Ansprüche 1 bis 8, dadurch gekennzeichnet, daß man die Umsetzung mit einem Dialkylcarbonat bei einer Temperatur von 80 bis 130°C durchführt.

11. Verfahren gemäß einem der Ansprüche 1 bis 10, dadurch gekennzeichnet, daß man die Reaktion des Oxims mit der Base in Gegenwart von Toluol, Xylol, Methanol, Ethanol oder Dimethylcarbonat durchführt.

12. Verfahren gemäß einem der Ansprüche 1 bis 11, dadurch gekennzeichnet, daß man die Umsetzung mit einem Dialkylcarbonat in einem Druckbereich von 1 bis 100 bar durchführt.

13. Verfahren gemäß Anspruch 12, dadurch gekennzeichnet, daß man die Umsetzung in Gegenwart von 0,1 bis 1 Moläquivalent Base durchführt.

## Claims

1. A process for preparing oxime ethers of the general formula Ia 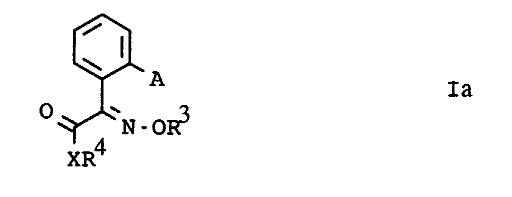 where
R³, R⁴ are, identically or differently, C₁-C₆-alkyl and R⁴ is additionally hydrogen, and
X is oxygen and NH, and
A is the following radicals:
CH₃, O-aryl, CH₂-O-Aryl, 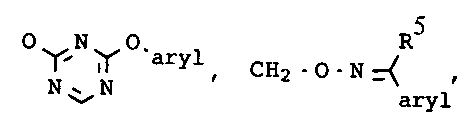 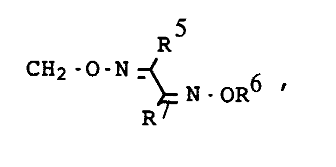 where
R⁵-R⁷ are, identically or differently, hydrogen, C₁-C₄-alkyl, aryl and hetaryl, with the proviso that
aryl
is phenyl and naphthyl and can be substituted by from one to three of the following radicals: halogen, cyano, nitro, C₁-C₄-alkyl, C₁-C₄-alkoxy, C₁-C₄-haloalkyl, C₁-C₄-haloalkoxy, C₁-C₄-alkoximino-C₁-C₄-alkyl, aryl, aryloxy, benzyl, benzyloxy, hetaryl, hetaryloxy, C₃-C₆-cycloalkyl, C₁-C₄-dialkylamino, CO₂CH₃, CO₂C₂H₅, formyl and acetyl,
and that hetaryl
is an unsubstituted or substituted aromatic five- or six-membered heterocycle,
which comprises converting an oxime of the general formula IIa 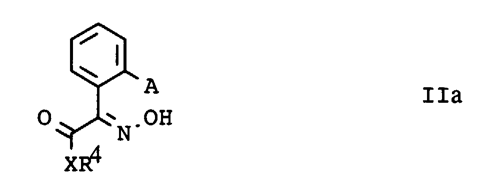 where the substituents have the abovementioned meanings, in the presence or absence of an organic diluent, with a base into the corresponding salt, and reacting the latter with a dialkyl carbonate of the general formula III 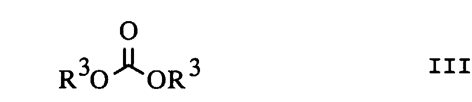 where R³ has the abovementioned meanings.

2. A process as claimed in claim 1, wherein an oxime of the general formula IIb, where the substituents have the abovementioned meanings, 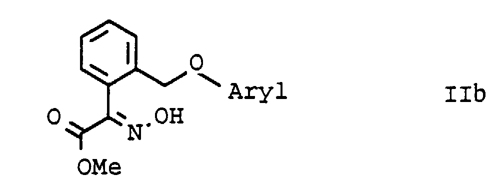 is first converted with sodium methanolate into the corresponding sodium salt, and the latter is then reacted with dimethyl carbonate to give the corresponding oxime ether.

3. A process as claimed in claim 1, wherein the compound IIc 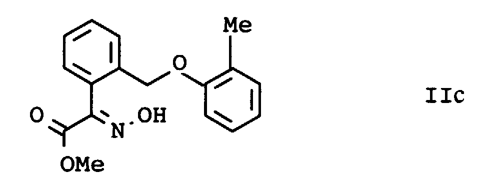 is converted with sodium methanolate into the corresponding sodium salt, and the latter is subsequently reacted with dimethyl carbonate to give the oxime ether Ib.

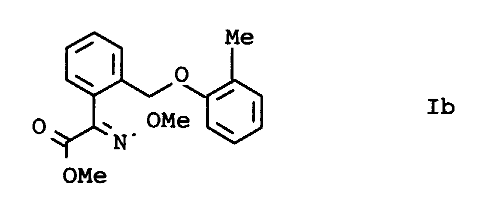

4. A process as claimed in claim 1, wherein R³ and R⁴ are methyl and X is oxygen.

5. A process as claimed in claim 1, wherein a tertiary amine or an alkali metal hydroxide, carbonate, alcoholate or hydride is used as base.

6. A process as claimed in claim 5, wherein triethylamine, trimethylamine or diethylmethylamine is used as base.

7. A process as claimed in any of claims 1 to 6, wherein from 0.1 to 3 mole equivalents of base are employed.

8. A process as claimed in any of claims 1 to 7, wherein dimethyl carbonate, diethyl carbonate, di-n-propyl carbonate, diisopropyl carbonate, di-n-butyl carbonate, di-n-pentyl carbonate or di-n-hexyl carbonate is used as dialkyl carbonate.

9. A process as claimed in any of claims 1 to 8, wherein the reaction of the oxime with the base is carried out at from 20 to 100°C.

10. A process as claimed in any of claims 1 to 8, wherein the reaction with a dialkyl carbonate is carried out at from 80 to 130°C.

11. A process as claimed in any of claims 1 to 10, wherein the reaction of the oxime with the base is carried out in the presence of toluene, xylene, methanol, ethanol or dimethyl carbonate.

12. A process as claimed in any of claims 1 to 11, wherein the reaction with a dialkyl carbonate is carried out under a pressure in the range from 1 to 100 bar.

13. A process as claimed in claim 12, wherein the reaction is carried out in the presence of from 0.1 to 1 mole equivalent of base.

## Revendications

1. Procédé pour la préparation d'éthers d'oximes de formule générale la 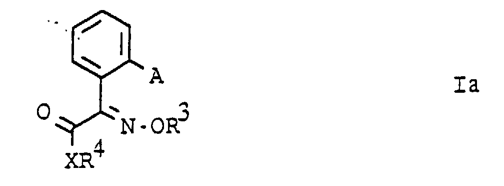 dans laquelle
R³, R⁴, ayant des significations identiques ou différentes, représentent chacun un groupe alkyle en C1-C6, R⁴ pouvant en outre représenter l'hydrogène et
X représente l'oxygène ou NH, et
A représente l'un des groupes suivants :
CH₃, O-Aryle CH₂-O-Aryle, 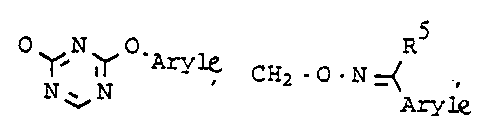 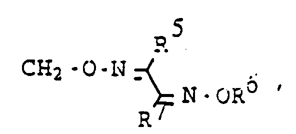 dans lesquels
R⁵ à R⁷, ayant des significations identiques ou différentes, représentent chacun l'hydrogène, un groupe alkyle en C1-C4, aryle ou hétéroaryle, sous réserve que
le groupe aryle
est un groupe phényle ou naphtyle et peut porter un à trois des substituants suivants : halogéno, cyano, nitro, alkyle en C1-C4, alcoxy en C1-C4, halogénoalkyle en C1-C4, halogénoalcoxy en C1-C4, (alcoximino en C1-C4)-alkyle en C1-C4, aryle, aryloxy, benzyle, benzyloxy, hétéroaryle, hétéroaryloxy, cycloalkyle en C3-C6, di-(alkyle en C1-C4)-amino, CO₂CH₃, CO₂C₂H₅, formyle ou acétyle,
et le groupe hétéroaryle
est un hétérocycle aromatique à cinq ou six chaînons, éventuellement substitué,
caractérisé par le fait que l'on convertit une oxime de formule générale IIa 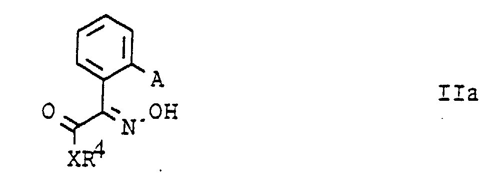 dans laquelle les symboles ont les significations indiquées ci-dessus, éventuellement en présence d'un diluant organique, à l'aide d'une base, en le sel correspondant qu'on fait réagir avec un carbonate de dialkyle de formule générale III 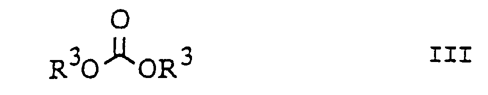 dans laquelle R³ a les significations indiquées ci-dessus.

2. Procédé selon la revendication 1, caractérisé par le fait que l'on convertit d'abord une oxime de formule générale IIb dans laquelle les symboles ont les significations indiquées ci-dessus, 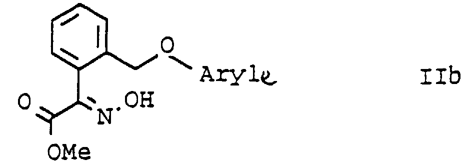 à l'aide du méthylate de sodium, en le sel de sodium correspondant qu'on fait réagir avec le carbonate de diméthyle, ce qui donne l'éther d'oxime correspondant.

3. Procédé selon la revendication 1, caractérisé par le fait que l'on convertit le composé IIc 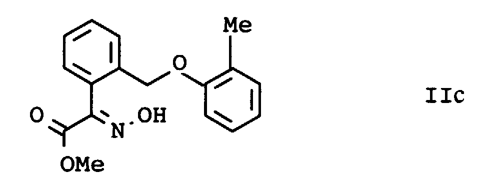 à l'aide du méthylate de sodium, en le sel de sodium correspondant qu'on fait ensuite réagir avec le carbonate de diméthyle, ce qui donne l'éther d'oxime Ib

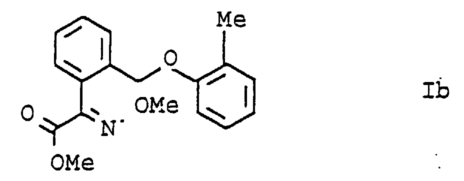

4. Procédé selon la revendication 1, caractérisé par le fait que R³ et R⁴ représentent des groupes méthyle et X l'oxygène.

5. Procédé selon la revendication 1, caractérisé par le fait que la base utilisée est une amine tertiaire ou un hydroxyde, carbonate, alcoolate ou hydrure alcalin.

6. Procédé selon la revendication 5, caractérisé par le fait que la base utilisée est la triéthylamine, la triméthylamine ou la diéthylamine.

7. Procédé selon l'une des revendications 1 à 6, caractérisé par le fait que l'on utilise la base en quantités de 0,1 à 3 équivalents molaires.

8. Procédé selon une des revendications 1 à 7, caractérisé par le fait que l'on utilise, en tant que carbonate de dialkyle, le carbonate de diméthyle, le carbonate de diéthyle, le carbonate de di-n-propyle, le carbonate de di-isopropyle, le carbonate de di-n-butyle, le carbonate de di-n-pentyle ou le carbonate de di-n-hexyle.

9. Procédé selon une des revendications 1 à 8, caractérisé par le fait que la réaction entre l'oxime et la base est réalisée à une température de 20 à 100°C.

10. Procédé selon une des revendications 1 à 8, caractérisé par le fait que la réaction avec un carbonate de dialkyle est réalisée à une température de 80 à 130°C.

11. Procédé selon l'une des revendications 1 à 10, caractérisé par le fait que la réaction entre l'oxime et la base est réalisée en présence de toluène, de xylène, de méthanol, d'éthanol ou de carbonate de diméthyle.

12. Procédé selon l'une des revendications 1 à 11, caractérisé par le fait que la réaction avec un carbonate de dialkyle est réalisée sous une pression de 1 à 100 bar.

13. Procédé selon la revendication 12, caractérisé par le fait que la réaction est réalisée en présence de 0,1 à 1 équivalent molaire de la base.
